# EUROPÄISCHE PATENTSCHRIFT

(11) **EP 0 186 732 B1**
(45) Veröffentlichungstag und Bekanntmachung des Hinweises auf die Patenterteilung: **03.02.1993**
(21) Anmeldenummer: 85111379.5
(22) Anmeldetag: 09.09.1985
(51) Int. Cl.: A61B 8/06

(54) **Ultraschall-Gerät zur Determination des peripheren Blutströmungsverhaltens**
Ultrasonic determination of peripheral blood flow behaviour
Appareil ultrasonique pour la détermination du comportement du flux sanguin périphérique

(30) Priorität: 21.09.1984 DE 3434740
(43) Veröffentlichungstag der Anmeldung: 09.07.1986
(73) Patentinhaber: Mauser, Rudolf, D-65510 Idstein (DE)
(72) Erfinder: Mauser, Rudolf, D-65510 Idstein (DE)
(74) Vertreter: Kraus, Walter, Dr.

(56) Entgegenhaltungen:
- EP-A- 0 016 399
- EP-A- 0 075 284
- EP-A- 0 102 733
- EP-A- 0 107 172
- AT-B- 343 265
- DE-A- 2 406 630
- DE-A- 2 461 264
- DE-A- 3 325 453

## Beschreibung

Die Erfindung betrifft ein Diagnosegerät zum Detektieren eines Blutflußsignals durch Messen der zeitlichen Veränderung der Strömung von Erythrozyten in praeterminalen und/oder terminalen Blutstrombahnen, umfassend:
(a) eine Ultraschall-Verschiebungsfrequenz-Meßeinrichtung zum Messen von Ultraschall-Verschiebungsfrequenzen, die durch Wechselwirkung der Erythrozyten mit einem Ultraschallfeld hervorgerufen werden;
(b) eine Einrichtung zum Entfernen des turbulenten Strömungsanteils der Erytrhozyten aus dem von der Ultraschall-Verschiebungsfrequenz-Meßeinrichtung ermittelten Ultraschall-Verschiebungsfrequenzspektrum, welche einen ersten und zweiten Demodulator aufweist, von denen der erste Demodulator die am Ausgang einer Ultraschallsende- und -empfangssonde vorhandenen elektrischen Verschiebungsfrequenzsignale bei einer ersten Phasenverschiebung, vorzugsweise von 0°, zum elektrischen Sendefrequenzsignal demoduliert, während der zweite Demodulator die am Ausgang der Ultraschallsende- und -empfangssonde vorhandenen Verschiebungsfrequenzsignale bei einer zweiten Phasenverschiebung zum elektrischen Sendefrequenzsignal demoduliert, welche vorzugsweise 90° beträgt;
(c) eine Filtereinrichtung zum Ausfiltern eines vorbestimmten Ultraschall-Verschiebungsfrequenzbereichs aus dem Ultraschall-Verschiebungsfrequenzspektrum; und
(d) eine Integrier- und Vergleichseinrichtung zum Bilden des Integrals der spektralen Verteilungsdichte der Ultraschall-Verschiebungsfrequenzen des vorbestimmten Ultraschall-Verschiebungsfrequenzbereichs und zum größenmäßigen Vergleich der von der Integrier- und Vergleichseinrichtung aufgrund zweier zeitlich nacheinander ermittelter Ultraschall-Verschiebungsfrequenzspektren gebildeten Integrale sowie zur Abgabe eines das Vergleichsergebnis repräsentierenden Diagnosesignals.

Dieses Diagnosegerät gestattet das Detektieren eines vaskulär autonomen Signals, durch welches es ermöglicht wird, das periphere Blutströmungsverhalten, insbesondere in der praeterminalen und terminalen Strombahn der Fingerbeere, als einen äußerst sensitiven Parameter zur Beschreibung der Kreislaufsituation eines Menschen, insbesondere eines Patienten, meßtechnisch zu erfassen.

Ein Diagnosegerät der vorstehend angegebenen grundsätzlichen Art ist aus der DE-A-3 325 453 bekannt, und zwar weist dieses Diagnosegerät eine Einrichtung zum Messen der zeitlichen Veränderung der Strömungsvektoren von Erythrozyten in Blutstrombahnen auf und ist prinzipiell für praeterminale und/oder terminale Blutstrombahnen einsetzbar, wobei bei kleineren Blutgefäßen nur eine Stelle im Blutgefäß beobachtet wird, und es ist dazu geeignet, Blutflußänderungen aufgrund evozierter extrakorporaler Stimulation zu ermitteln.

Man hat schon seit langem versucht, den von P. Nogier entdeckten RAC-Effekt (Reflex-Auriculocardinal-Effekt) bzw. den VAS-Effekt (Vaskuläres-Autonomes-Signal-Effekt) meßtechnisch zuverlässig zu erfassen.

Aufgabe der Erfindung ist es insbesondere, ein Diagnosegerät der obigen Art zu schaffen, das es ermöglicht, den RAC- bzw. VAS-Effekt instantan und quantitativ zu detektieren.

Diese Aufgabe wird mit einem Diagnosegerät der eingangs genannten Art dadurch gelöst, daß die Einrichtung zum Entfernen des turbulenten Strömungsanteils der Erythrozyten folgendes umfaßt:
(1) einen dritten Demodulator, welcher die am Ausgang der Ultraschallsende- und -empfangssonde vorhandenen Verschiebungsfrequenzsignale bei einer dritten Phasenverschiebung zum elektrischen Sendefrequenzsignal demoduliert, welche vorzugsweise 180° beträgt;
(2) eine Summiereinrichtung, welche die von den Demodulatoren abgegebenen Demodulationssignale summiert; und
(3) eine Vergleichseinrichtung, welche aus den Summen der Demodulationssignale und den Differenzen der Demodulationssignale Differenzsignale bildet und das jeweils größte dieser Differenzsignale auf ihren Ausgang abgibt.

Dieses Diagnosegerät arbeitet nach einem Meßverfahren, das die Blutströmungsgeschwindigkeit und deren vektorielles Strömungsverhalten in der Arteria radialis unter Einfluß extrakoporaleer Stimulation determiniert.

Das am Meßort registrierte Signal resultiert aufgrund der Widerstandsveränderungen in den praeterminalen und terminalen Strombahnen der Fingerbeere und kann aufgrund entsprechender Kenntnis der Physiologie und Hämodynamik diagnostisch interpretiert werden.

Der außerordentlich komplizierte Regelmechanismus der Mikrozirkulation sowie die individuelle und optimale Anpassung an den jeweiligen Stoffwechselbedarf schließt eine allgemein gültige Beschreibung dieses Gefäßabschnittes von vornherein aus. Die mit dem Diagnosegerät nach der Erfindung durchzuführenden Messungen beziehen sich vorzugsweise auf die distalen oberen Extremitäten.

Bei den Untersuchungen, die im Rahmen der vorliegenden Erfindung durchgeführt wurden, hat es sich aufgrund der meßtechnischen und medizinischen Untersuchungsergebnisse sowie unter Zugrundelegung des tatsächlichen Entstehungsgeschehens der obigen Effekte gezeigt, daß die Bezeichnung RAC-Effekt ebenso wie die Bezeichnung VAS-Effekt sachlich nicht zutreffend ist, sondern daß die strömungsdynamische Veränderung von Erythrozytenvektoren entscheidend ist, die mit dem Diagnosegerät nach der Erfindung durch ein Meßsignal detektiert wird.

Primär wird diese strömungsdynamische Veränderung von Erythrozytenvektoren zwar durch die Regelmechanismen der praeterminalen Strombahn herbeigeführt, der detektierte Effekt, ob manuell oder elektronisch, ist jedoch eine vektorielle Änderung im Blutströmungsverhalten. Dementsprechend soll dieser Effekt im Rahmen der vorliegenden Beschreibung als EVI-Effekt bezeichnet werden, wobei EVI die Abkürzung für die englische Bezeichnung "Evoked Velocity Interference", d.h. "evozierte Geschwindigkeitsinterferenz", ist. Dieser EVI-Effekt wird mit dem Diagnosegerät nach der Erfindung detektiert, indem als EVI-Signal die zeitliche Veränderung der Strömungsvektoren von Erythrozyten aufgrund evozierter extrakorporaler Stimulation gemessen wird. Obzwar derzeit noch lange nicht alle diagnostischen Auswertungsmöglichkeiten des EVI-Effekts erforscht sind, steht außer Zweifel, daß der EVI-Effekt vorhanden ist, bei Gesunden wie Kranken ausgelöst werden kann und sein Zustandekommen wissenschaftlich fundiert interpretierbar ist.

Als Ultraschallfrequenzen werden insbesondere die Frequenzen von 8 bis 25 MHz, vorzugsweise von 10 bis 25 MHz, besonders bevorzugt von 15 bis 25 MHz, verwendet.

Die Einrichtung zum Entfernen des turbulenten Strömungsanteils der Erythrozyten, die hier auch als Einrichtung zum Entfernen des durch die Rotation der Erythrozyten hervorgerufenen Anteils aus dem von der Ultraschall-Verschiebungsfrequenz-Meßeinrichtung ermittelten Ultraschall-Verschiebungsfrequenzspektrum bezeichnet wird, und die Filtereinrichtung zum Ausfiltern eines vorbestimmten Ultraschall-Verschiebungsfrequenzbereichs aus dem Ultraschall-Verschiebungsfrequenzspektrum dienen dazu, alle für die Auflösung, d.h. für die optimale Ermittlung der zeitlichen Veränderung der Strömungsvektoren der Erythrozyten störenden Frequenzen aus den von der Ultraschall-Verschiebungsfrequenz-Meßeinrichtung erfaßten Ultraschall-Verschiebungsfrequenzen auszuscheiden. Es können zwar sowohl im Turbulenz- bzw. Rotationsanteil als auch in den ausgeschiedenen Frequenzen noch gewisse Signalanteile sein, die im Prinzip zur Bildung des Diagnosesignals beitragen könnten, aber dieser Signalanteil ist so geringwertig, daß er ohne weiteres weggelassen werden kann.

Vorzugsweise ist ein Monitor zur Wiedergabe des zeitlichen Verlaufs der Differenz der Integrale der spektralen Verteilungsdichte der Ultraschall-Verschiebungsfrequenzen des vorbestimmten Ultraschall-Verschiebungsfrequenzbereichs an die Integrier- und Vergleichseinrichtung angekoppelt. Auf diese Weise kann der Benutzer das Auftreten und den genauen Verlauf des Diagnosesignals jeweils auf dem Monitor verfolgen, wobei der Monitor vorzugsweise ein Fernseh- bzw. Bildröhrenmonitor oder ein Kurvenschreiber ist, jedoch auch eine sonstige Kurvendarstellungseinrichtung sein kann.

Bevorzugt ist das mit einem Monitor in der vorstehend genannten Weise ausgerüstete Diagnosegerät weiterhin so ausgebildet, daß der Monitor außerdem zur, vorzugsweise gleichzeitigen, Wiedergabe des zeitlichen Verlaufs der über die Zeit gemittelten spektralen Verteilungsdichte der Ultraschall-Verschiebungsfrequenzen des vorbestimmten Ultraschall-Verschiebungsfrequenzbereichs mit einer Spektralverteilungsdichtebestimmungseinrichtung verbunden ist, welche mit ihrem Eingang an den Ausgang der Filtereinrichtung und mit ihrem Ausgang an den Eingang der Integriereinrichtung angekoppelt ist.

Auf diese Weise kann auf dem Monitor sowohl der zeitliche Verlauf der Differenz der Integrale der spektralen Verteilungsdichte der Ultraschall-Verschiebungsfrequenzen als auch der zeitliche Verlauf der über die Zeit gemittelten spektralen Verteilungsdichte dieser Ultraschall-Verschiebungsfrequenzen synchron, zum Beispiel übereinander, dargestellt werden, so daß also die Zeitdarstellungsachsen übereinstimmen und der eine dieser beiden Verläufe im oberen Teil der Monitorkurvendarstellungsfläche erscheint, während der andere Verlauf im unteren Teil der Monitorkurvendarstellungsfläche erscheint. Die zusätzlich zur Wiedergabe des zeitlichen Verlaufs der Differenz der Integrale der spektralen Verteilungsdichte erfolgende Wiedergabe des zeitlichen Verlaufs der über die Zeit gemittelten spektralen Verteilungsdichte, insbesondere in Form einer sogenannten Hüllkurve, hat, abgesehen von der synchronen Zuordnung der beiden Verläufe, vor allem den Vorteil, daß die Darstellung der über die Zeit gemittelten spektralen Verteilungsdichte eine visuelle Kontrolle ermöglicht, ob ein hämodynamisch akzeptables Ultraschall-Verschiebungsfrequenzsignal vorliegt, das nachstehend auch als Δf-Signal bezeichnet ist, und ob die Sondenapplikation am Patienten so erfolgt ist, daß das zu erfassende Blutgefäß genau von der Sonde getroffen wird.

In einer Weiterbildung der Erfindung kann das Diagnosegerät vorteilhaft so ausgebildet sein, daß die Filtereinrichtung sowohl direkt als auch über einen ersten Speicher an die Spektraldichteverteilungsbestimmungseinrichtung angekoppelt ist, und daß die Spektraldichteverteilungsbestimmungseinrichtung über einen zweiten Speicher an die Integrier- und Vergleichseinrichtung und den Monitor angekoppelt ist.

Diese Ausbildung ermöglicht es, in dem ersten Speicher einen Bezugswert des Δf-Signals, d.h. also Bezugs-Ultraschall-Verschiebungsfrequenzen zu speichern, wie sie beispielsweise während eines Herzschlags des Patienten erhalten werden, wenn noch keine extrakorporale Stimulation evoziert ist, so daß die Spektraldichteverteilungsbestimmungseinrichtung einerseits eine über die Zeit gemittelte spektrale Bezugsverteilungsdichte aufgrund des Speicherinhalts des ersten Speichers berechnen und in den zweiten Speicher eingeben kann, und daß die Spektraldichteverteilungsbestimmungseinrichtung außerdem laufend während einer gewissen Zeit, die eine Vielzahl von Herzschlägen umfassen kann, aktuelle über die Zeit gemittelte Spektraldichteverteilungen bestimmt und ebenfalls in den zweiten Speicher eingibt, aus dem die aktuellen und die Bezugswerte entnommen und von der Integriereinrichtung verarbeitet werden können. Außerdem können dem Monitor die entsprechenden aktuellen Werte für die Darstellung der über die Zeit gemittelten Spektraldichteverteilung zugeführt werden.

Es sei hier darauf hingewiesen, daß es natürlich auch möglich ist, daß die Integrier- und Vergleichseinrichtung den Bezugswert dem ersten Speicher entnimmt und die aktuellen Werte aus dem zweiten Speicher erhält, wozu dann die Integrier- und Vergleichseinrichtung allerdings den Bezugswert aus dem ersten Speicher noch in eine über die Zeit gemittelte spektrale Verteilungsdichte umrechnen müßte.

Obwohl die aus der Filtereinrichtung erhaltenen Ultraschall-Verschiebungsfrequenzen im Prinzip analog verarbeitet und gespeichert werden könnten, ist es zu bevorzugen, abgesehen von einer vorzugsweise analogen Darstellung auf dem Monitor, die Ultraschall-Verschiebungsfrequenzen bis einschließlich der Integrierung der über die Zeit gemittelten spektralen Verteilungsdichte, die Signalverarbeitung digital durchzuführen, wozu in die Verbindung zwischen der Filtereinrichtung einerseits und dem ersten Speicher sowie der Spektraldichteverteilungsbestimmungseinrichtung andererseits ein Analog-Digital-Umsetzer eingefügt ist, und wozu vorzugsweise, d.h. zum Zwecke der analogen Darstellung auf dem Monitor in die Verbindung zwischen dem zweiten Speicher und dem Monitor sowie in die Verbindung zwischen der Integrier- und Vergleichseinrichtung und dem Monitor ein Digital-Analog-Umsetzer eingefügt ist.

Außerdem kann das Diagnosegerät nach der Erfindung eine herzzyklussynchrone Triggerimpulse erzeugende Steuereinrichtung aufweisen, die an die Integrier- und Vergleichseinrichtung, die Speicher und die Spektraldichteverteilungsbestimmungseinrichtung angekoppelt ist und jeweils einen periodischen Betrieb dieser Baueinheiten, wie er beispielsweise weiter unten in näheren Einzelheiten erläutert ist, veranlaßt.

Außerdem kann das erfindungsgemäße Diagnosegerät in Weiterbildung der Erfindung ergänzt sein durch eine die spektrale Verteilungsdichte der Ultraschall-Verschiebungsfrequenzen ermittelnde Frequenzanalysiereinrichtung. Diese Frequenzanalysiereinrichtung ermöglicht es, den im jeweiligen Fall interessierenden Frequenzbereich, der durch die Filtereinrichtung durchgelassen werden soll, zu ermitteln.

Diese Frequenzanalysiereinrichtung zeichnet sich in einer bevorzugten Ausführungsform aus durch eine den Ausgang der Ultraschall-Verschiebungsfrequenz-Meßeinrichtung mit dem Ausgang einer Steuereinrichtung zum Erzeugen eines herzzyklussynchronen Triggerimpulses variabler Zeitdauer verknüpfende Torschaltung, und einen an die Torschaltung angekoppelten Mehrkanalanalysator zur Mehrkanalanalyse des durch die Torschaltung durchgelassenen Frequenzbereichs der Ultraschall-Verschiebungsfrequenzen. Auf diese Weise ist es möglich, aus dem Herzzyklus einen bestimmten gewünschten Bereich auszuwählen.

Im einzelnen kann dieser Teil der Frequenzanalysiereinrichtung so ausgebildet sein, daß die Steuereinrichtung an eine Elektrokardiogrammeinheit angekoppelt ist und in Hintereinanderschaltung folgendes aufweist:
(a) eine Triggerimpulserzeugungsschaltung zum Erzeugen eines herzzyklussynchronen Triggerimpulses vorbestimmter Zeitdauer;
(b) eine Triggerimpulsverzögerungs- oder -verlängerungsschaltung; und
(c) eine durch den verzögerten Triggerimpuls oder die Abstiegsflanke des verlängerten Triggerimpulses getriggerte Impulserzeugungseinrichtung, die Impulse einstellbarer Länge erzeugt.

Die vorstehenden sowie weitere Vorteile und Merkmale seien nachfolgend anhand einiger in den Figuren der Zeichnung im Prinzip dargestellter, besonders bevorzugter Ausführungsformen der Erfindung näher erläutert; es zeigen:
- Figur 1: eine perspektivische Darstellung einer Ultraschallsonde eines erfindungsgemäßen Diagnosegeräts;
- Figur 2: eine schematische Darstellung der in näheren Einzelheiten ausgeführten Ultraschallsonde der Figur 1, in dem Zustand, in dem sie an eine Arteria radialis eines zu untersuchenden Patienten angekoppelt ist;
- Figuren 3A und 3B: Blockschaltbilder von bevorzugten Ausführungsformen des Diagnosegeräts nach der Erfindung;
- Figur 4: eine Blockschaltbilddarstellung einer Ausführungsform einer Einrichtung zur Ausschaltung des turbulenten Strömungsanteils der Erythrozyten bzw. des durch die Rotation der Erythrozyten hervorgerufenen Anteils aus dem von der Ultraschall-Verschiebungsfrequenz-Meßeinrichtung des Diagnosegeräts ermittelten Ultraschall-Verschiebungsfrequenzspektrum;
- Figur 5: ein Blockschaltbild einer Ausführungsform einer Frequenzanalysiereinrichtung, mit der es möglich ist, den interessierenden Frequenzbereich zu ermitteln, auf welchen der Durchlaßbereich der Filtereinrichtung des Diagnosegeräts einzustellen ist; und
- Figur 6: eine Kurvendarstellung zur Erläuterung der Betriebsweise einer in der Frequenzanalysiereinrichtung nach Figur 5 vorgesehenen Triggerimpulserzeugungseinrichtung.

Das in den Figuren dargestellte auf hochfrequentem Ultraschall basierende Meßverfahren und Diagnosegerät detektiert unter Ausnutzung des Doppler-Effekts die strömungsvektoriellen Veränderungen der Erythrozyten, die aufgrund einer evozierten Veränderung in der praeterminalen und terminalen Strombahn entstehen. Als Meßort wurde hierbei die Arteria radialis gewählt, deren Elastizität als intakt vorausgesetzt, eine additive and agglomerative Inversion der Erythrozytenströmungsvektoren unter dem Einfluß einer Stimulation zum Beispiel im Bereich der Concha ergibt. Der eigentliche Ort des Geschehens liegt distal in der praeterminalen Strombahn der Fingerbeeren und sollte theoretisch auch dort gemessen werden. Hierbei würde sich einerseits zwar die Latenzzeit verkürzen und die Empfindlichkeit deutlich erkennbar ansteigen, andererseits läßt jedoch eine einzelne Fingerbeere keine repräsentative Aussage über das Blutströmungsverhalten und dessen Regulationsmechanismus in der Extremität zu. Zwangsweise muß hier ein Kompromiss geschlossen werden, so daß die Arteria radialis für die Routineuntersuchung eine medizinsich hinreichende Akzeptanz gewährleistet.

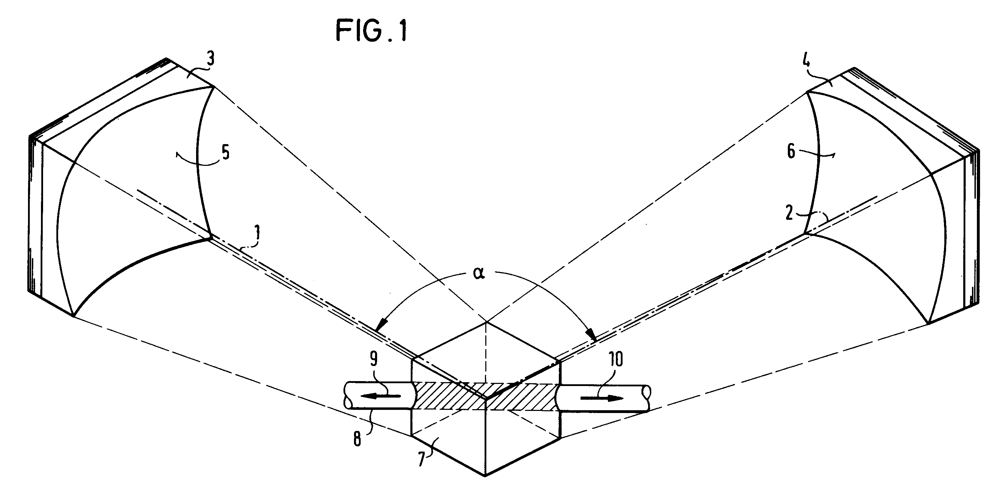

In der Figur 1 ist die Wandlerkonfiguration einer bevorzugten Ultraschallsonde für das Diagnosegerät gezeigt. In dieser Wandlerkonfiguration sind die Achsen 1 und 2 der beiden piezoelektrischen Wandlerelemente 3 und 4 unter einem Winkel α von bevorzugt 45° angeordnet. Die piezoelektrischen Wandlerelemente 3 und 4 haben eine derart gewölbte Strahlungs- bzw. Empfangsoberfläche 5 bzw. 6, daß ihr Strahlungs- bzw. Empfangsbereich fokussierend längs der Achse 1 bzw. 2 verläuft. Eines der beiden piezoelektrischen Wandlerelemente 3 oder 4 wird wahlweise als Ultraschallsender verwendet, während das andere dieser beiden piezoelektrischen Wandlerelemente 4 oder 3 als Ultraschallempfänger verwendet wird. Die beiden Achsen 1 und 2 schneiden sich im Probenvolumen 7, das schematisch durch einen gedachten Kubus angedeutet ist, durch welchen das zu untersuchende Blutgefäß, also vorliegend die Arteria radialis 8, hindurchgeht. Es sei, obwohl die piezoelektrischen Wandlerelemente 3 und 4 in ihrer Ultraschallsende- und -empfangsfunktion gegeneinander ausgetauscht werden können, hier davon ausgegangen, daß das piezoelektrische Wandlerelement 3 der Ultraschallsender und das piezoelektrische Wandlerelement 4 der Ultraschallempfänger ist, unter dieser Voraussetzung stellt der mit 9 bezeichnete Pfeil den vom Ultraschallempfänger weggewandten Strömungshauptvektor der in der Arteria radialis 8 fließende Erythrozyten dar, während der mit 10 bezeichnete Pfeil den zum Ultraschallempfänger hin gerichteten Strömungshauptvektor dieser Erythrozyten darstellt (TOWARDS- bzw. AWAY-Vektor).

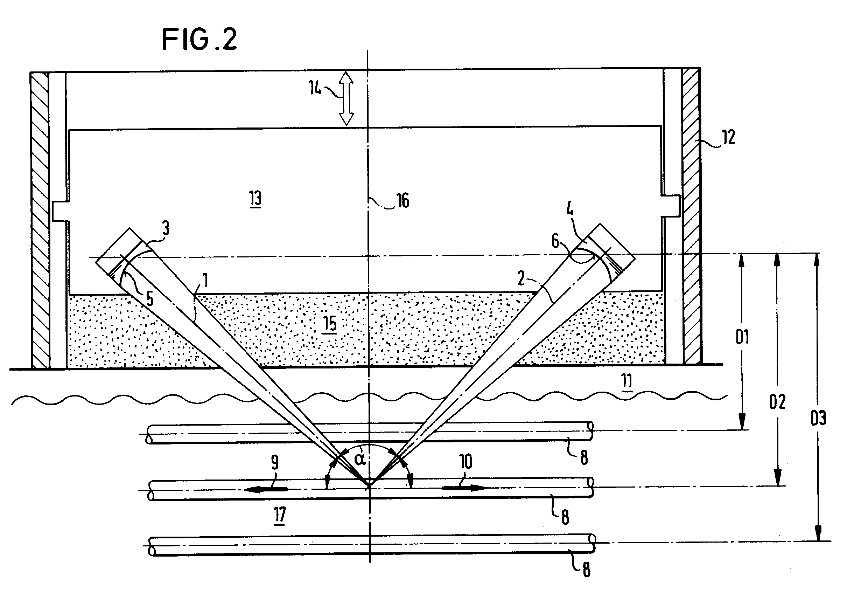

In Figur 2 ist ein Schnitt durch den gesamten Ultraschallmeßkopf gezeigt. Hiernach sind die piezoelektrischen Wandlerelemente 3 und 4 in einem Ultraschallkopfgehäuse 12 auf einem Träger 13 angebracht, der, wie durch den Doppelpfeil 14 angedeutet ist, senkrecht zur Oberfläche der Haut 11 verschieb- und damit einstellbar ist. Zwischen der Haut 11 und dem Träger 13 bzw. den piezoelektrischen Wandlerelementen 3 und 4 befindet sich ein Koppelmedium 15, zum Beispiel ein Koppelgel.

In der Figur 2 sind mehrere mögliche Positionen der Arteria radialis angedeutet, wobei die mittlere Entfernung D2 zwischen der Achse der Arteria radialis und den Mittelpunkten der Strahlungs- bzw. Empfangsoberflächen 5 und 6 der piezoelektrischen Wandlerelemente 3 bzw. 4 in der Praxis etwa 5 mm beträgt und das kubische Probenvolumen 7 (siehe Figur 1) vorzugsweise zu etwa 2 mm³ gewählt wird. Der Schnittpunkt der beiden Achsen 1 und 2 kann auf der Ultraschallkopfachse 16, die senkrecht zur Oberfläche der Haut 11 verläuft, durch Verstellung des Trägers 13 um eine gewisse Einstellstrecke von vorzugsweise ± 3 mm verstellt werden, d.h. auf die Entfernungen D1 und D3 in Figur 2.

Nach Durchtritt der Ultraschallstrahlungs- bzw. -empfangskeule des piezoelektrischen Wandlerelements 3 bzw. 4 verlaufen diese Ultraschallstrahlungs- bzw. -empfangskeulen divergent, und die Absorption des Ultraschalls nimmt quadratisch mit der Entfernung zu.

Die sinusoide Applikationsfrequenz des Ultraschalls beträgt in der vorliegenden Ausführungsform etwa 15 MHz und wird mittels eines piezoelektrischen Wandlerelements aus Bleizirkonat-Titanat in Longitudinalwellen gleicher Frequenz umgewandelt sowie mit einer mittleren Leistung von 4 mW/cm² (calorimetrisch gemessen) in den Untersuchungsbereich 17 eingeschallt.

Die verwendete Ultraschallfrequenz liegt allgemein im Bereich von 8 bis 25 MHz, vorzugsweise im Bereich von 10 bis 25 MHz, und besonders bevorzugt im Bereich von 15 bis 25 MHz.

Die mittlere eingestrahlte Ultraschalleistung sollte möglichst klein sein, um eine biologische Schädigung auszuschließen, sie sollte also höchstens 50 mW/cm² (calorimetrisch gemessen) betragen. Im Hinblick auf das mögliche Auftreten einer lokalen Hyperämisierung, d.h. Durchblutungsförderung, durch Wärmeeinfluß und eine hierdurch möglich Meßwertverfälschung, ist es jedoch zu bevorzugen, die eingestrahlte Ultraschalleistung im Bereich von 2 bis 10 mW/cm² (calorimetrisch gemessen) zu halten, da man in diesem Bereich einerseits ausreichend gute und deutliche Meßwerte erhält und andererseits keine Meßwertverfälschung zu erwarten ist.

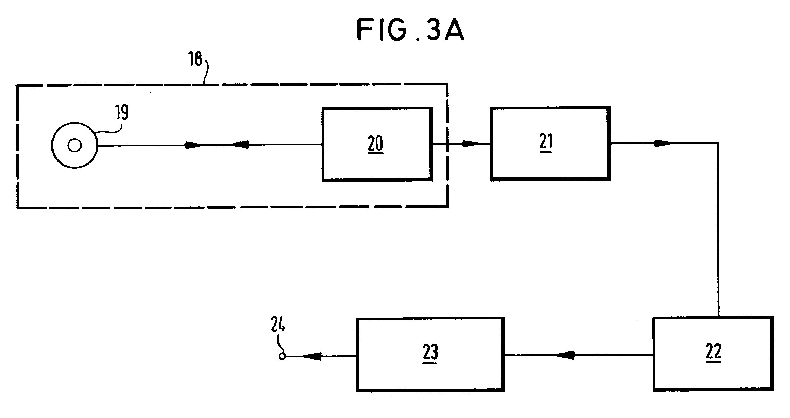

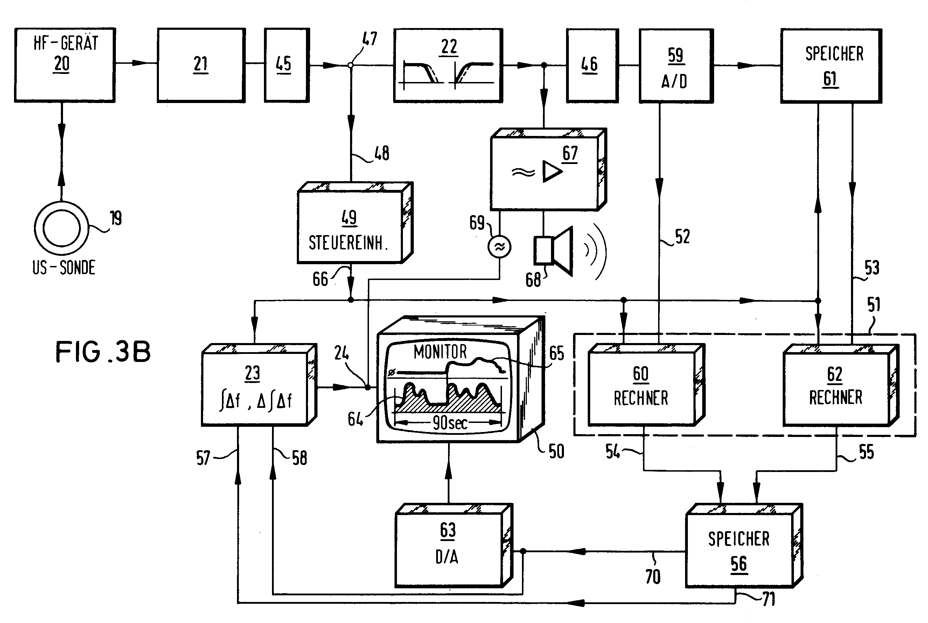

Es sei nun auf das in Figur 3 dargestellte Blockschaltbild einer bevorzugten Ausführungsform eines Diagnosegeräts näher eingegangen, mit dem das hier vorgeschlagene EVI-Meßverfahren verwirklicht wird:
Dieses Diagnosegerät besteht in der in Figur 3 gezeigten Ausführungsform im wesentlichen aus einer Einrichtung zum Messen der zeitlichen Veränderung der Strömungsvektoren von Erythrozyten in praeterminalen und/oder terminalen Blutstrombahnen aufgrund evozierter extrakorporaler Stimulation.

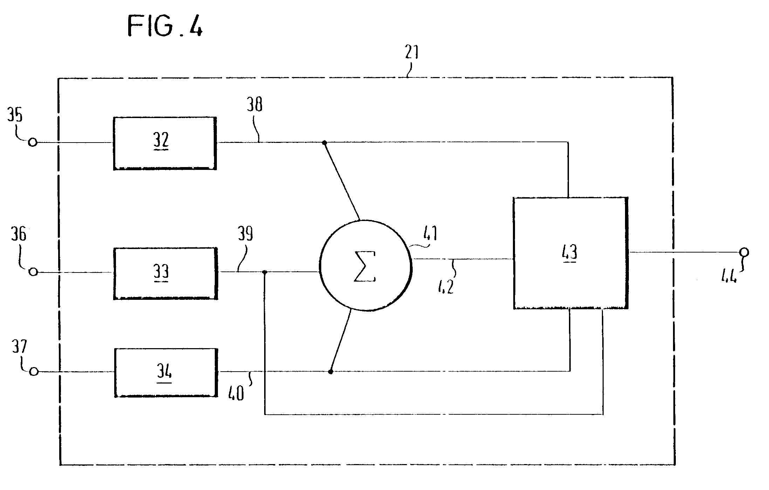

Im einzelnen weist das in Figur 3A in einer Grundausführung dargestellte Diagnosegerät folgendes auf:
eine Ultraschall-Verschiebungsfrequenz-Meßeinrichtung 18 zum Messen von Ultraschall-Verschiebungsfrequenzen, die durch Wechselwirkung der Erythrozyten mit einem Ultraschallfeld hervorgerufen werden. Diese Ultraschall-Verschiebungsfrequenz-Meßeinrichtung 18 umfaßt nach der Figur 3A eine Ultraschallsonde bzw. einen Ultraschallkopf 19, beispielsweise wie in den Figuren 1 und 2 dargestellt, sowie eine Hochfrequenzsende- und -empfangseinrichtung 20, welche mit der Ultraschallsonde 19 verbunden ist und diese betreibt. Diese Hochfrequenzsende- und -empfangseinrichtung 20 ist mit einer Einrichtung 21 zum Entfernen des durch die Rotation der Erythrozyten hervorgerufenen Anteils aus dem von der Ultraschall-Verschiebungsfrequenz-Meßeinrichtung 18 ermittelten Ultraschall-Verschiebungsfrequenzspektrum verbunden. Diese Einrichtung ist weiter unten in näheren Einzelheiten erläutert und enthält insbesondere eine Demodulatoreinrichtung zum Demodulieren der eingegebenen Hochfrequenzsignale. Der Einrichtung 21 ist eine Filtereinrichtung 22 zum Ausfiltern eines vorbestimmten Ultraschall-Verschiebungsfrequenzbereichs aus dem Ultraschall-Verschiebungsfrequenzspektrum nachgeschaltet. Die Filtereinrichtung 22 kann bei der Ausführungsform der Einrichtung 21 nach Figur 4 aus einem Filter bestehen und die endgültig zu verarbeitenden Verschiebungsfrequenzsignale, die sogenannten Δf-Signale, können vor oder hinter der Filtereinrichtung 22 auf einer gemeinsamen Leitung geführt werden.

Der Filtereinrichtung 22 ist eine Integrier- und Vergleichseinrichtung 23 nachgeschaltet, die das Integral der über die Zeit gemittelten spektralen Verteilungsdichte der Ultraschall-Verschiebungsfrequenzen des durch die Filtereinrichtung 22 durchgelassenen vorbestimmten Ultraschall-Verschiebungsfrequenzbereichs bildet und einen größenmäßigen Vergleich der aufgrund zweier zeitlich nacheinander ermittelten Ultraschall-Verschiebungsfrequenzspektren gebildeten Integrale durchführt sowie ein das Ergebnis dieses Vergleichs repräsentierendes Diagnosesignal an ihrem Ausgang 24 abgibt.

Eine Einrichtung 21 zum Entfernen des durch die Rotation der Erythrozyten hervorgerufenen Anteils aus dem von der Ultraschall-Verschiebungsfrequenz-Meßeinrichtung 18 ermittelten Ultraschall-Verschiebungsfrequenzspektrum, die in Figur 4 dargestellt ist, umfaßt einen ersten Demodulator 32, einen zweiten Demodulator 33 und einen dritten Demodulator 34, welche an ihren Eingängen 35, 36 und 37 jeweils das Sendefrequenzsignal f₀ und das Empfangsfrequenzsignal f₀ + Δf in elektrischer Form erhalten, wozu natürlich die Eingänge 35, 36 und 37 jeweils aus zwei Leitungen bestehen. In dem der Blutgeschwindigkeit proportionalen Empfangssignal f₀ + Δf repräsentiert Δf eine Vielzahl von Verschiebefrequenzen. Dieses Empfangssignal wird in einem Breitbandverstärker aufbereitet und den Demodulatoren neben dem Sendesignal f₀ zugeführt.

Der Demodulator 32 führt eine Demodulation mit einer Phasenverschiebung von φ =0° gegenüber dem Sendefrequenzsignal f₀ durch und gibt daher an seinem Ausgang 38 alle diejenigen Frequenzen aus dem Gemisch f₀ ± Δf ab, die gleich oder kleiner als die Sendefrequenz sind. Der Demodulator 33 führt eine Demodulation mit φ=90° gegenüber dem Sendefrequenzsignal f₀ durch und gibt daher an seinem Ausgang 39 von dem Frequenzgemisch f₀ ± Δf alle diejenigen Frequenzen ab, die gleich oder größer als die Sendefrequenz sind. Schließlich führt der Demodulator 34 eine Demodulation mit φ =180° gegenüber dem Sendefrequenzsignal f₀ durch und gibt daher aus dem Frequenzgemisch f₀ ± Δf alle Frequenzen in zeitlicher Reihenfolge ab.

Die Ausgänge 38, 39 und 40 der Demodulatoren 32, 33 und 34 sind an eine Summiereinrichtung 41 angeschlossen, welche die von den Demodulatoren abgegebenen Demodulationssignale summiert und das Summensignal über deren Ausgang 43 an eine Vergleichseinrichtung 43 gibt, die vorzugsweise ein Rechner ist und die außerdem direkt an die Ausgänge 38, 39 und 40 der Demodulatoren 32, 33 und 34 angekoppelt ist.

Die Vergleichseinrichtung 43 arbeitet in folgender Weise:
(a) sie bildet ein erstes sekundäres Differenzsignal, das gleich der Differenz zwischen der Summe der Demodulationssignale einerseits und der Differenz zwischen den Ausgangssignalen der Demodulatoren 32 und 33 andererseits ist;
(b) sie bildet ein zweites sekundäres Differenzsignal, das gleich der Differenz zwischen der Summe der Demodulationssignale einerseits und der Differenz zwischen den Ausgangssignalen der Demodulatoren 33 und 34 andererseits ist;
(c) sie bildet ein drittes sekundäres Differenzsignal, das gleich der Differenz zwischen der Summe der Demodulationssignale einerseits und der Differenz zwischen den Ausgangssignalen der Demodulatoren 32 und 34 andererseits ist; und
(d) sie gibt immer das jeweils größte dieser sekundären Differenzsignale auf ihren Ausgang 44.

Auf diese Weise erhält man am Ausgang 44 bereits ein Frequenzgemisch mit stochastischer Verteilungsdichte, welches frei von dem durch die Rotation der Erythrozyten hervorgerufenen Anteil ist und nach einer Niederfrequenzsignalaufbereitung gegebenenfalls nach Durchlaufen eines Regelverstärkers in die Filtereinrichtung 22 eingegeben werden kann.

Die Filtereinrichtung 22 umfaßt im Falle der Verwendung einer Einrichtung 21 der in Figur 4 gezeigten Art in Hintereinanderschaltung ein Hochpaßfilter, dessen Güte Q und dessen Frequenz f_{c} vorzugsweise durch ein binärkodiertes Signal über Tasten programmierbar sind. Bevorzugt ist ein Festprogramm mit einer oberen Grenzfrequenz F_{HI} von 480 Hz bzw. von einer Zeitkonstanten v von 0,00208 sec oder mit einer oberen Grenzfrequenz von 960 Hz bzw. von einer Zeitkonstante τ von 0,00104 sec des Hochpaßfilters vorgesehen. Dem Hochpaßfilter ist ein Tiefpaßfilter nachgeschaltet, ln dem gemäß dem vorgesehenen Festprogramm eine Abtrennung aller Frequenzanteile entweder oderhalb von 1250 Hz entsprechend einer Zeitkonstanten τ von 0,0008 sec oder oberhalb von 2100 Hz entsprechend einer Zeitkonstanten τ von 0,00047 sec erfolgt. Gemäß diesem Festprogramm können also vier bevorzugte Frequenzfenster bzw. -Durchlaßbereiche der Filtereinrichtung 22 ausgewählt werden, nämlich 480 bis 1250 Hz, 480 bis 2100 Hz, 960 bis 1250 Hz und 960 bis 2100 Hz. Außerdem können andere Frequenzdurchlaßbereiche der Filtereinrichtung 22 bevorzugt manuell oder durch andere Programme eingestellt werden.

Es sei an dieser Stelle erwähnt, daß diese Grenzfrequenzen bzw. -zeitkonstanten der Filtereinrichtung 22 in einem direkten Bezug zum Rotationsverhalten der Erythrozyten bei manipulierter Vasomotion der praeterminalen Blutstrombahn stehen, und durch die vorgenannte umschaltbare Filtereinrichtung 22 stehen zur rechnerischen Prüfungsauswertung jeweils vier autonome Frequenzfenster zur Verfügung. Die Selektion des jeweilig zur Berechnung, also Weiterverarbeitung,verwendeten Frequenzbereichs ist primär von der augenblicklichen Kreislaufsituation des Patienten sowie von dessen Gefäß- und Durchblutungsstatus abhängig und muß von Fall zu Fall individuell vorgenommen werden. Als Kriterium hierfür kann die weiter unten näher erläuterte, auf dem unteren Teil des Monitors in Figur 3B angedeutete Δf-Hüllkurve sowie ein Display aus einer Reihe lichtemittierenden Dioden dienen, das den jeweiligen vertikalen Endpunkt der Δf-Hüllkurve anzeigt. In beiden Fällen sollte die Programmierung der Filtereinrichtung 22 solange verändert werden, bis die diastolische/enddiastolische Strömungsgeschwindigkeit den kleinsten Wert aufweist.

In jedem Fall erhält man am Ausgang der Filtereinrichtung 22 das innerhalb des gewählten Frequenzbands erscheinende Verschiebungsfrequenzgemisch aus den verschiedenen Verschiebungsfrequenzen Δf, das dann der Integrier- und Vergleichseinrichtung 23, von der eine Ausführungsform weiter unten in Verbindung mit der Erläuterung der Figur 3B näher beschrieben ist, zugeführt wird.

Es sei nun die in Figur 3B in einem in nähere Einzelheiten gehenden Blockschaltbild dargestellte Ausführungsform eines Diagnosegeräts erläutert:

Auch die Ausführungsform der Figur 3B umfaßt, wie in dieser Figur eingezeichnet, die Ultraschallsonde 19, die Hochfrequenzsende- und -empfangseinrichtung 20, eine Einrichtung 21 zum Entfernen des durch die Rotation der Erythrozyten hervorgerufenen Anteils aus dem von der Ultraschall-Verschiebungsfrequenz-Meßeinrichtung 19, 20 ermittelten Ultraschall-Verschiebungsfrequenzspektrum, die beispielweise gemäß Figur 4A oder Figur 4B ausgebildet sein kann, und die Integrier- und Vergleichseinrichtung 23, an deren Ausgang 24 das Diagnosesignal erhalten wird. Im übrigen umfaßt diese Ausführungsform zusätzlich zur Figur 3A noch die nachfolgend näher beschriebenen Baueinheiten.

Zunächst ist eine Niederfrequenzsignalaufbereitungseinrichtung 45 vorgesehen, die im Falle der Verwendung einer Einrichtung 21 nach Figur 4 zwischen die Einrichtung 21 udn die Filtereinrichtung 22 geschaltet sein kann, wie in Figur 3B dargestellt.

Dem Ausgang der Filtereinrichtung 22 ist ein Regelverstärker 46 nachgeschaltet, der dazu dient, das innerhalb des von der Fitlereinrichtung 22 durchgelassenen Frequenzbandes auftretende Frequenzgemisch in seiner Amplitude zu normieren. Dieser Regelverstärker kann jedoch auch zwischen die Niederfrequenzsignalaufbereitungseinrichtung 45 und Filtereinrichtung 22 geschaltet werden, und zwar vor der Abzweigung 47, an der eine Leitung 48 abzweigt, die zum Eingang einer Steuereinheit 49 geht, in welchem Falle für die Steuereinheit 49 kein gesonderter Regelverstärker erforderlich ist.

Bevor auf die zwischen dem Regelverstärker 46 und der Integrier- und Vergleichseinrichtung 23 gemäß Figur 3B vorgesehenen Baueinheiten und deren Funktionsweise näher eingegangen wird, sei zunächst darauf hingewiesen, daß in der Ausführungsform nach Figur 3B ein Monitor 50 zur Wiedergabe des zeitlichen Verlaufs der Differenz der Integrale der spektralen Verteilungsdichte der Ultraschall-Verschiebungsfrequenzen des von der Filtereinrichtung 22 durchgelassenen vorbestimmten Ultraschall-Verschiebungsfrequenzbereichs an den Ausgang 24 der Integrier- und Vergleichseinrichtung angeschlossen ist. Diese vorgenannte Differenz der Integrale wird nachstehend auch als Δ∫Δf-Signal bezeichnet, während die einzelnen Integrale auch als ∫Δf-Signale bezeichnet werden. Das Δ∫Δf-Signal ist im vorliegenden Beispielsfall auf der oberen Hälfte des als Fernsehbildschirm-Monitor ausgebildeten Monitors 50 wiedergegeben, wie bei 65 dargestellt.

Der Monitor 50 ist außerdem zur gleichzeitigen Wiedergabe des zeitlichen Verlaufs der über die Zeit gemittelten spektralen Verteilungsdichte der Ultraschall-Verschiebungsfrequenzen des von der Filtereinrichtung 22 durchgelassenen vorbestimmten Ultraschall-Verschiebungsfrequenzbereichs mit einer Spektralverteilungsdichtebestimmungseinrichtung 51 verbunden. Diese ist mit ihrem Eingang 52 bzw. 53 über weiter unten erläuterte Baueinheiten und gegebenenfalls den Regelverstärker 46 an den Ausgang der Filtereinrichtung 22 angekoppelt. Der Ausgang 54 bzw. 55 ist über einen Speicher 56 an den Eingang 57 bzw. 58 der Integrier- und Vergleichseinrichtung 23 angekoppelt.

Die Ankopplung der Filtereinrichtung 22 an die Spektralverteilungsdichtebestimmungseinrichtung 51 umfaßt einen Analog-zu-Digital-Umsetzer 59, dessen Ausgangssignal direkt über den Eingang 52 in einen ersten Rechner 60 der Spektralverteilungsdichtebestimmungseinrichtung 51 gegeben wird. Außerdem ist der Ausgang des Analog-zu-Digital-Umsetzers 59 über einen Speicher 61 und den Eingang 53 mit einem zweiten Rechner 62 der Spektralverteilungsdichtebestimmungseinrichtung 51 verbunden. Schließlich ist der Ausgang des Speichers 56 über einen Digital-zu-Analog-Umsetzer 63 mit dem Monitor 50 zur Wiedergabe der über die Zeit gemittelten spektralen Verteilungsdichte auf dem Monitor verbunden. Diese über die Zeit gemittelte Spektralverteilungsdichte wird vorliegend im unteren Teil des Monitorbildschirms als Hüllkurve wiedergegeben, wie bei 64 angedeutet ist. Das hierzu in den Monitor eingegebene Signal wird nachfolgend auch als Δf-Hüllkurvensignal bezeichnet und wird vorliegend auf dem Monitorbildschirm zeitlich synchron mit dem Δ∫Δf-Signal wiedergegeben.

Zur Erstellung eines zentralen herzzyklussynchronen Steuersignals für die komplette Meßeinrichtung in der Steuereinheit 49 wird dieser Steuereinheit über die Leitung 48 entweder das Δf-Signal des Demodulationskanals mit φ = 0 oder das kombinierte Δf-Signal zugeführt. Sofern dieses Signal nicht bereits über den Regelverstärker 46 normiert worden ist, was von der Anordnung des Regelverstärkers, wie oben im einzelnen erläutert, abhängt, ist in der Stuereinheit 49 ein eigener Regelverstärker vorgesehen. Das mit dem Regelverstärker normierte Δf-Signal wird in der Steuereinheit 49 über einen Nulldurchgangsdetektor in ein impulsbreitenmoduliertes Signal umgesetzt. Nach Konvertierung dieses Signals durch einen Frequenz-Spannungs-Umsetzer, der ebenfalls in der Steuereinheit 49 vorgesehen ist, wird von dem daraus resultierenden Analogsignal durch Differenzierung die erste mathematische Ableitung (erster Differentialquotient) ermittelt und ein Triggersignal Sₜ erstellt, das am Ausgang 66 der Steuereinheit 49 erhalten wird. Dieses Triggersignal wird, wie in Figur 3B angedeutet ist, der Integrier- und Vergleichseinrichtung 23, den Rechnern 60 und 62 und dem Speicher 61 zugeführt und dient dazu, eine zeitlich synchrone Ablaufsteuerung dieser Baueinheiten zu bewirken.

Schließlich ist an den Ausgang der Filtereinrichtung 22 ein Niederfrequenzverstärker 67 angekoppelt, an dessen Ausgang ein Kopfhörer oder Lautsprecher 68 angeschlossen ist, wodurch die akustische Darstellung der Blutströmungsgeschwindigkeit und der deren Verhalten proportionalen Verschiebungsfrequenz erfolgen kann. Je nach der Ankopplung und Ausbildung des Niederfrequenzverstärkers 67 und des Kopfhörers oder Lautsprechers 68 kann die akustische Darstellung stereophon entsprechend den Strömungsvektoren 9 und 10 (siehe Figuren 1 und 2) erfolgen. Das dabei übertragene Frequenzband entspricht dem der oben erwähnten vier Frequenzfenster der Filtereinrichtung 22, sofern nach deren Festprogramm gearbeitet wird.

Schließlich ist an den Ausgang 24 der Integrier- und Vergleichseinrichtung 23 ein Oszillator 69 angekoppelt und mit dem Niderfrequenzverstärker 67 verbunden, so daß beim Auftreten eines Diagnosesignals, das hier auch als EVI-Signal bezeichnet ist und den Oszillator 69 in Gang setzt, dem im Kopfhörer oder Lautsprecher 68 hörbaren Strömungssignal ein Dauerton überlagert wird, der einen durch ein Diagnosesignal detektierten Effekt signalisiert.

Die grundsätzliche Funktionsweise des sich an den Ausgang der Filtereinrichtung 22 anschließenden Teils der Schaltung der Figur 3B ist folgende:
Im Speicher 61 wird aufgrund eines manuellen Befehls ein Bezugs-Δf-Signal gespeichert, was beispielsweise zu Beginn eines Meßzyklus erfolgen kann, wobei dieses Bezugs-Δf-Signal beispielsweise einem beliebigen Herschlag entspricht, der noch von einer evozierten extrakorporalen Stimulation unbeeinflußt ist. Dieses vorher durch den Analog-zuDigital-Umsetzer 59 digitalisierte Bezugs-Δf-Signal steht dem Rechner 62 zur Verfügung, welcher einen Bezugswert der zeitlich gemittelten spektralen Verteilungsdichte der Verschiebungsfrequenzen errechnet und diesen Bezugswert in den Speicher 56 eingibt.

Alle in der darauffolgenden Meßperiode von beispielsweise 90 Sekunden pro Herzschlag eintreffenden Δf-Signale werden nach Digitalisierung im Analog-zu-Digital-Umsetzer 59 als aktuelle Δf-Signale fortlaufend dem Rechner 60 zugeführt, der die über die Zeit gemittelten jeweils aktuellen spektralen Verteilungsdichten der Verschiebungsfrequenzen berechnet und ebenfalls in den Speicher 56 eingibt.

Infolgedessen erstellen beide Rechner 60 und 62 die über die Zeit gemittelte spektrale Verteilungsdichtekurve der Verschiebungsfrequenzen, wobei der Rechner 60 die aktuelle Verteilungsdichtekurve und der Rechner 62 eine Bezugsverteilungsdichtekurve erstellt. Die durch den Rechner 60 erstellten Verteilungsdichtekurven werden dem Speicher 56 über den Ausgang 70 entnommen, anschließend mittels des Digital-zuAnalog-Umsetzers 63 digitalisiert und dann auf dem Bildschirm des Monitors 50 als Hüllkurve 64 dargestellt, die sich über die Meßperiode von vorliegend 90 sec erstreckt, wie auf dem Bildschirm in Figur 3B angedeutet.

Außerdem wird sowohl die aktuelle über die Zeit gemittelte spektrale Verteilungsdichtekurve der Verschiebungsfrequenzen über den Ausgang 70 des Speichers 56 als auch die über die Zeit gemittelte spektrale Bezugsverteilungsdichtekurve der Verschiebungsfrequenzen über den Ausgang 71 des Speichers 56 den Eingängen 57 bzw. 58 der Integrier- und Vergleichseinrichtung 23 zugeführt, welche diese beiden Verteilungsdichtekurven integriert, also fortlaufend aktuelle Integral-Δf-Signale und ein Bezugs-Integral-Δf-Signal während der Meßperiode erstellt sowie fortlaufend während der Meßperiode die Differenz zwischen dem jeweils aktuellen Integral-Δf-Signal und dem Bezugs-Integral-Δf-Signal bildet, so daß man fortlaufend aktuelle Δ∫Δf-Signale erhält, die insgesamt über die Meßperiode hinweg auf dem Bildschirm des Monitors 50 eine Δ∫Δf-Kurve 65 ergeben. Wenn die beiden Signale, aus denen das jeweils aktuelle Δ∫Δf-Signal durch Differenzbildung erzeugt wird, nicht mehr als 1% voneinander abweichen, wird das Δ∫Δf-Signal zu Null angenommen, so daß dadurch das Rauschen unterdrückt und eine rauschfreie glatte Nullinie innerhalb Identität der erwähnten Signale im Bereich von ≦1% auf dem Bildschirm des Monitors 50 geschrieben wird. Wie bereits weiter oben erwähnt, kann der Monitor auch eine andere Darstellungs- und/oder Aufzeichnungseinrichtung, beispielsweise ein ZweiKanal-Recorder sein. Bevorzugt werden sowohl die Δf-Hüllkurvensignale als auch die Δ∫Δf-Signale zusätzlich auf zwei voneinander unabhängigen LED-Bargraph-Displays als oszillierende Balken oder Punkte dargestellt, so daß dadurch die trägheitslose Überwachung des Blutströmungsverhaltens ermöglicht wird. Die Berechnung von einem der beiden Strömungsvektoren 9 oder 10 kann im übrigen von der bevorzugten Ausführungsform wahlweise per Taste veranlaßt werden.

Eine besonders bevorzugte Ausführungsform der Integrier- und Vergleichseinrichtung 23 besitzt folgenden Aufbau:
Sie besteht im wesentlichen aus einem Spannungs-Frequenz-Umsetzer mit großer Bandbreite, einer Start-Stop-Einheit und einem Bit-Zähler mit Speicher, insbesondere einem 4 x 4 Bit-Zähler.

In dieser Integrier- und Vergleichseinrichtung wird das pulsatile Δf-Hüllkurvensignal über den Spannungsfrequenzumsetzer in ein amplitudenkonstantes Rechtecksignal umgewandelt, dessen Frequenz um so höher ist, je größer die Amplitude der Hüllkurve wird (du/df). Das Ausgangssignal des Spannungs-Frequenz-Umsetzers ist somit direkt proportional der Eingangsamplitude und weist einen Hub von 10 kHz/1 V auf, so daß 1 V einem Δf-Wert von 1000 Hz entspricht. Hieraus ergibt sich für die Bandbreite eine Auflösung von 10 Impulsen pro Hz, was bedeutet Δf = 1 mV in der Δf-Hüllkurve. Beim Eintreffen des ersten herzzyklussynchronen Triggerimpulses von der Steuereinheit 49 werden über die Start-Stop-Einheit der im 4 x 4 Bit-Zähler der Integrier- und Vergleichseinrichtung 23 vorhandenen Werte gelöscht, und der Zählereingang wird für eine vorbestimmte Zeitdauer, die vorzugsweise 450 msec beträgt, zur Dateneinlesung freigegeben. Nach Ablauf dieser Dateneinlesungsperiode erfolgt ein Datentransfer in den Speicher der Integrier- und Vergleichseinrichtung 23 und ein Löschen des 4 x 4 Bit-Zählers. Der dimensionslose Speicherwert repräsentiert das Flächenintegral der Δf-Hüllkurve und wird als Bezugswert verwendet. Da die Berechnung des aktuellen Δ∫Δf-signals vorzugsweise von Herzschlag zu Herzschlag durchgeführt wird, erfolgt in diesem Falle die Ergebnisdarstellung um einen Herzzyklus verschoben.

Da bei der Stimulation, zum Beispiel im Bereich der Concha, sowohl eine Zunahme als auch eine Abnahme der Blutströmungsgeschwindigkeit im Meßareal gegenüber dem Ausgangswert auftreten kann, muß die Berechnung der ∫Δf-Werte unter Berücksichtigung eines Polaritäts-Vorzeichens erfolgen. Die Basislinie des Monitors, beispielsweise eines Recorders befindet sich deshalb in der Mitte und weist in beiden Richtungen einen graduierten Bereich von beispielsweise 25 Gradteilungen für 250 Hz der errechneten Δf-Werte auf. Über ein digitales Vorwahlprogramm wird dem Rechner der Integrier- und Vergleichseinrichtung 23 zum Beispiel entsprechend 250 Hz der errechneten Δf-Werte eingegeben und eine Subtraktion in Bezug auf die Anzeige durchgeführt. Das heißt, unabhängig von der autonom ablaufenden instantanen Berechnung kann die Darstellung dahingehend beeinflußt werden, daß der eingegebene Wert von errechneten subtrahiert und erst dann angezeigt wird, wenn dieser überschritten wird.

Da bei der nachträglichen Befundung ersichtlich sein muß, mit welchem Subtraktionswert operiert wurde, erfolgt bei Überschreiten des vorgewählten Wertes am Monitor bzw. Recorder eine Sprungfunktion in der Höhe des eingegebenen Wertes. In praxi würde sich zum Beispiel für den Wert "5" eine Sprungfunktion von 12.5 Gradteilungen = 125 Hz des errechneten Δf-Werts ergeben. Steigt die Strömungsgeschwindigkeit weiter an, so zeigt der am Monitor bzw. Recorder dargestellte Wert so lange einen analogen Verlauf, bis der eingestellte Sollwert unterschritten wird und mit einer Sprungfunktion auf die Basislinie zurückkehrt. Analoges gilt für den Vorgang mit umgekehrten Polaritätsvorzeichen. Parallel hierzu kann über ein LED-Display eine Vorzeichenanzeige des EVI- bzw. Diagnose-Signals erfolgen.

Das am Bargraph-Display dargestellt ∫Δf-Signal unterliegt nicht dieser Rechenopertaion und zeigt somit jede Veränderung mit entsprechendem Polaritätsvorzeichen an, soweit sich eine Abweichung gegenüber dem gespeicherten Bezugswert ergibt. Durch die Gegenregulation in der praeterminalen Strombahn besteht nach Beendigung der Stimulation noch einige Zeit ein verändertes Strömungsverhalten in Form von Nachschwankungen, die ein gegenüber dem Ausgangswert veränderts Flächenintegral ergeben. Ist diese Gegenregulation, die bis zu einer Minute andauern kann, bis auf ± 10% gegenüber dem gespeicherten Wert abgesunken, kann eine z.B. grüne LED-Anzeige signalisieren, daß nunmehr ein neuer Bezugswert im Speicher 61 eingespeichert werden kann. Erfolgte nämlich vor diesem Zeitpunkt die Eingabe eines neuen Bezugswerts, dann wäre die nachfolgende Berechnung zwangsweise falsch positiv.

Es sei darauf hingewiesen, daß die einzelnen Rechnerdaten über einen digitalen Videomonitor mit Bildspeicher abgerufen und unter Zwischenschaltung eines Modulators auf einem Fernsehmonitor zur Darstellung gebracht werden können.

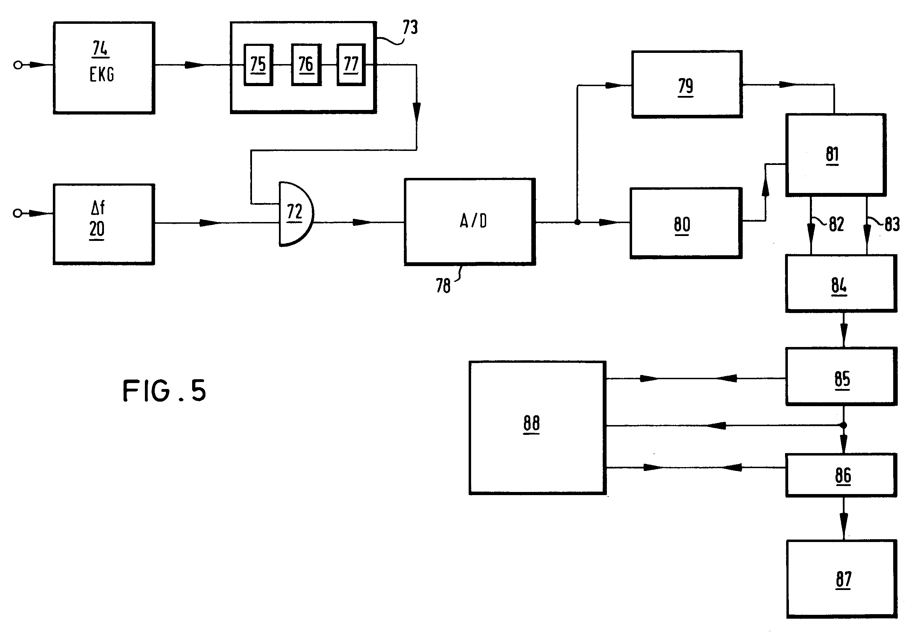

Es sei nun auf Figur 5 Bezug genommen, die ein Blockschaltbild einer bevorzugten Frequenzanalysiereinrichtung zur Ermittlung der spektralen Verteilungsdichte der Ultraschall-Verschiebungsfrequenzen zeigt, welche es ermöglicht, den jeweils interessierenden Frequenzbereich, auf den die Filtereinrichtung 22 einzustellen ist, festzustellen. In dieser Frequenzanalysiereinrichtung kann das vollständige Δf-Signal, das vor der Filtereinrichtung 22 abgenommen wird, im Frequenzbereich von vorzugsweise 0 bis 3 kHz, also dem gesamten Verschiebungsfrequenzbereich, in einer Mehrzahl von Kanälen, zum Beispiel in 256 Kanälen, auf spektrale Häufigkeitsverteilung untersucht werden. Diese spektrale Häufigkeitsverteilung kann beispielsweise durch eine Histogramm von Blaken oder Punkten dargestellt werden. Die in den einzelnen Kanälen, beispielsweise in den 256 Kanälen, ermittelten Werte werden vorzugsweise digitalisiert in einen Speicher gegeben.

Im einzelnen ist die Frequenzanalysiereinrichtung nach Figur 5 wie folgt aufgebaut:
Das Δf-Signal kommt von der Ultrachall-Verschiebungsfrequenz-Meßeinrichtung 20 entweder direkt oder über die Einrichtung 21 und/oder 45 (siehe insbesondere Figur 3B) und wird über eine Torschaltung 72 mit dem Ausgangssignal einer Steuereinrichtung 73 verknüpft, die ihr Eingangssignal von einer Elektrokardiogrammeinheit 74 erhält. Die Steuereinrichtung 73 dient zum Erzeugen eines variablen herzzyklussynchronen Triggerimpulses und umfaßt in Hintereinanderschaltung folgende Baueinheiten: eine Triggerimpulserzeugungsschaltung 75 zum Erzeugen eines herzzyklussynchronen Triggerimpulses vorbestimmter Zeitdauer, sowie eine Triggerimpulsverzögerungs- oder -verlängerungsschaltung 76 und eine durch den verzögerten Triggerimpuls oder die Abstiegsflanke des verlängerten Triggerimpulses getriggerte Impulserzeugungseinrichtung 77, die Impulse einstellbarer Länge erzeugt.

Der an den Ausgang der Torschaltung 72 angekoppelte Teil der Frequenzanalysiereinrichtung umfaßt, wie die Figur 5 zeigt, folgendes:
Einen Analog-zu-Digital-Umsetzer 78, der zwischen den Ausgang der Torschaltung 72 und die parallelen Eingänge eines Bezugsspeichers 79 undeines Signalspeichers 80 geschaltet ist. Sowohl der Bezugsspeicher 79 als auch der Signalspeicher 80 sind mit den Eingängen eines Rechners 81 verbunden, dessen Ausgänge 82 und 83 an den Eingang eines Multiplexers angeschlossen sind. Dem Multiplexer 84 ist ein Mehrkanalfilter 85 nachgeschaltet, das beispielsweise ein 256 Kanalfilter sein kann. Auf dieses Mehrkanalfilter 85 folgt ein Videoumsetzer 86, dem ein Monitor 87 nachgeschaltet ist. Mit dem Mehrkanalfilter 85 und dem Videoumsetzer 86 sowie mit der Verbindung zwischen dem Mehrkanalfilter und dem Videoumsetzer 86 kann ein Punkt-Histogrammrechner 88 verbunden sein.

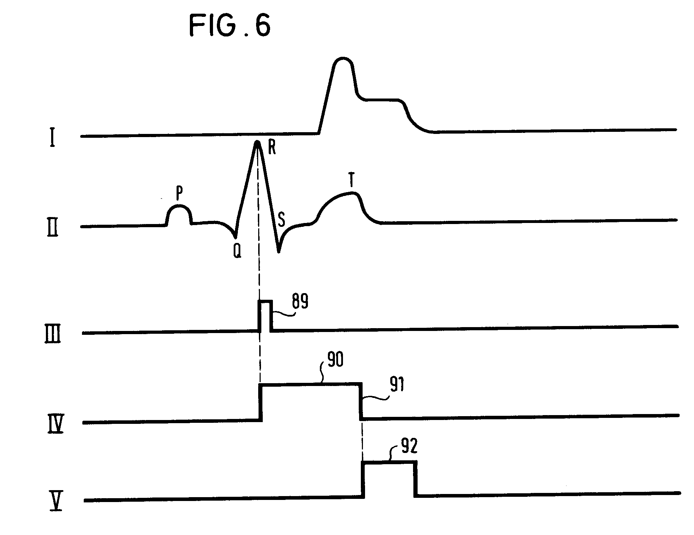

In Figur 6 sind einige Kurven zur Erläuterung der Arbeitsweise des Eingangsteils der Frequenzanalysiereinrichtung nach Figur 5 dargestellt, und zwar stellt die Kurve I den zeitlichen Verlauf des Δf-Signals dar, während die Kurve II zeitlich synchron den Verlauf eines Elektrokardiogramms zeigt; die Kurve III zeigt einen in der Triggerimpulserzeugungsschaltung 75 erhaltenen Triggerimpuls; die Kurve IV zeigt den durch die Triggerimpulsverzögerungs- bzw. -verlängerungsschaltung 76 verlängerten Triggerimpuls; und die Kurve V zeigt den von der Abstiegsflanke des verlängerten Triggerimpulses ausgelösten Impuls, der von der Impulserzeugungseinrichtung 77 abgegeben wird und in seiner Länge einstellbar ist.

Im einzelnen arbeitet die Steuereinrichtung 73 der Figur 5 wie folgt:
Die Triggerimpulserzeugungsschaltung 75 leitet einen herzzyklussynchronen Triggerimpuls 89 aus dem EKG-Signal der Herzsystole, nämlich dem QRS-Komplex, ab. Die nachgeschaltete Triggerimpulsverzögerungs- bzw. -verlängerungsschaltung 76 verlängert den Triggerimpuls 89 variabel einstellbar bis über den gesamten Bereich der Diastole, so daß sich der Impuls 90 in Figur 6 ergibt. Die Impulserzeugungsschaltung 77 kann beispielsweise eine monostabile Torschaltung sein, welche mit der Abstiegsflanke 91 das Impulses 90 geöffnet wird und ein hochliegendes Signal im Bereich einer zeitlichen Länge von 0 bis 450 msec abgibt, d.h. bis über die gesamte Länge der hämodynamischen Phase. Diese Länge ist einstellbar, und man erhält den Impuls 92 in Figur 6. Das Ausgangssignal der Steuereinrichtung 73 öffnet die Torschaltung 21, so daß eine gewisser Frequenzbereich aus dem Gesamt-Δf-Bereich für die Auswertung durchgelassen wird, oder bei maximaler Länge des Steuerimpulses auch der Gesamt-Δf-Bereich. Diese durchgelassenen Frequenzen werden in dem Analog-zu-Digital-Umsetzer 78 digitalisiert, und es wird im Bezugsspeicher 79 ein repräsentativer Herzzyklus ohne Stimulation des Patienten gespeichert, während in den Signalspeicher 80 das aufgrund einer Stimulation, Provokation, Evokation veränderte Δf-Signal des gleichen Zeitbereichs (positionsmäßig und längenmäßig), wie es das Δf-Signal des Bezugssepichers 79 ist, eingegeben wird. Der Rechner 81 berechnet die Differenz aus den den einzelnen Frequenzen entsprechenden Häufigkeiten. Der eine Ausgang 82 des Rechners ermöglicht es, wahlweise den Inhalt des Bezugsspeichers 79 oder des Signalspeichers 80 in den Multiplexer 84 einzugeben, während über den Ausgang 83 des Rechners die vorgenannten Differenzen in den Multiplexer 84 eingegeben werden. Der Multiplexer 84 schaltet die eingegebenen Frequenzen synchron ablaufend durch, so daß am Ausgang des nachgeschalteten Mehrkanalfilters 85 gewichtete Kanäle erhalten werden (häufigkeitsgewichtet).

Der Punkt-Histogrammrechner 88 bewirkt über einen Cursor die wahlweise Ausschnittsdarstellung einer aufgenommenen spektralen Verteilung, so daß entweder eine Punktdarstellung oder eine histographische erhalten wird, und zwar entweder über den vollen Δf-Bereicn oder über einen Ausschnitt des Gesamt-Δf-Bereichs. Die von der Verbindungsstelle zwischen dem Mehrkanalfilter 85 und dem Videoumsetzer 86 zum Punkt-Histogrammrechner 88 in Figur 5 eingezeichnete Leitung führt das erforderliche Steuersignal zum Cursor zu.

Durch diese Frequenzanalysiereinrichtung können, wie bereits erwähnt, interessierende Frequenzbereiche ermittelt und in die Filtereinrichtung 22 der Figuren 3A und 3B einprogrammiert werden.

## Patentansprüche

1. Diagnosegerät zum Detektieren eines Blutflußsignals durch Messen der zeitlichen Veränderung der Strömung von Erythrozyten in praeterminalen und/oder terminalen Blutstrombahnen, umfassend:
(a) eine Untraschall-Verschiebungsfrequenz-Meßeinrichtung (19, 20) zum Messen von Ultraschall-Verschiebungsfrequenzen, die durch Wechselwirkung der Erythrozyten mit einem Ultraschallfeld hervorgerufen werden;
(b) eine Einrichtung (21) zum Entfernen des turbulenten Strömungsanteils der Erythrozyten aus dem von der Ultraschall-Verschiebungsfrequenz-Meßeinrichtung (19, 20) ermittelten Ultraschall-Verschiebungsfrequenzspektrum, welche einen ersten und zweiten Demodulator (32, 33) aufweist, von denen der erste Demodulator (32) die am Ausgang einer Ultraschallsende- und -empfangssonde (29) vorhandenen elektrischen Verschiebungsfrequenzsignale bei einer ersten Phasenverschiebung, vorzugsweise von 0°, zum elektrischen Sendefrequenzsignal demoduliert, während der zweite Demodulator (33) die am Ausgang der Ultraschallsende- und -empfangssonde (19) vorhandenen Verschiebungsfrequenzsignale bei einer zweiten Phasenverschiebung zum elektrischen Sendefrequenzsignal demoduliert, welche vorzugsweise 90° beträgt;
(c) eine Filtereinrichtung (22) zum Ausfiltern eines vorbestimmten Ultraschall-Verschiebungsfrequenzbereichs aus dem Ultraschall-Verschiebungsfrequenzspektrum; und
(d) eine Integrier- und Vergleichseinrichtung (23) zum Bilden des Integrals der spektralen Verteilungsdichte der Ultraschall-Verschiebungsfrequenzen des vorbestimmten Ultraschall-Verschiebungsfrequenzbereichs und zum größenmäßigen Vergleich der von der Integrier- und Vergleichseinrichtung (23) aufgrund zweier zeitlich nacheinander ermittelter Ultraschall-Verschiebungsfrequenzspektren gebildeten Integrale sowie zur Abgabe eines das Vergleichsergebnis repräsentierenden Diagnosesignals;
dadurch **gekennzeichnet,** daß die Einrichtung (21) zum Entfernen des turbulenten Strömungsanteils der Erythrozyten folgendes umfaßt:
(1) einen dritten Demodulator (34), welcher die am Ausgang der Ultraschallsende- und -empfangssonde (19) vorhandenen Verschiebungsfrequenzsignale bei einer dritten Phasenverschiebung zum elektrischen Sendefrequenzsignal demoduliert, welche vorzugsweise 180° beträgt;
(2) eine Summiereinrichtung (41), welche die von den Demodulatoren (32, 33, 34) abgegebenen Demodulationssignale summiert; und
(3) eine Vergleichseinrichtung (43), welche aus den Summen der Demodulationssignale und den Differenzen der Demodulationssignale Differenzsignale bildet und das jeweils größte dieser Differenzsignale auf ihren Ausgang (42) abgibt.

2. Diagnosegerät nach Anspruch 1, dadurch **gekennzeichnet,** daß die Vergleichseinrichtung (43) ein Rechner ist.

3. Diagnosegerät nach Anspruch 1 oder 2, **gekennzeichnet** durch einen Monitor (50), der zur Wiedergabe des zeitlichen Verlaufs der Differenz der Integrale der spektralen Verteilungsdichte der Ultraschall-Verschiebungsfrequenzen des vorbestimmten Ultraschall-Verschiebungsfrequenzbereichs an die Integrier- und Vergleicheinrichtung (23) angekoppelt ist.

4. Diagnosegerät nach Anspruch 3, dadurch **gekennzeichnet,** daß der Monitor (50) außerdem zur, vorzugsweise gleichzeitigen, Wiedergabe des zeitlichen Verlaufs der über die Zeit gemittelten spektralen Verteilungsdichte der Ultraschall-Verschiebungsfrequenzen des vorbestimmten Ultraschall-Verschiebungsfrequenzbereichs mit einer Spektralverteilungsdichtebestimmungseinrichtung (51) verbunden ist, die zwei Rechner (60, 62) umfaßt, welche mit ihrem Eingang (52, 53) an den Ausgang der Filtereinrichtung (22) und mit ihrem Ausgang (54, 55) an den Eingang (57, 58) der Integrier- und Vergleichseinrichtung (23) angekoppelt sind.

5. Diagnosegerät nach Anspruch 4, dadurch **gekennzeichnet,** daß die Filtereinrichtung (22) sowohl direkt als auch über einen ersten Speicher (61) an die Spektraldichteverteilungsbestimmungseinrichtung (51) angekoppelt ist, und daß die Spektraldichteverteilungsbestimmungseinrichtung (51) über einen zweiten Speicher (56) an die Integrier- und Vergleichseinrichtung (23) und den Monitor (50) angekoppelt ist.

6. Diagnosegerät nach Anspruch 5, dadurch **gekennzeichnet,** daß in die Verbindung zwischen der Filtereinrichtung (22) einerseits und dem ersten Speicher (61) sowie der Spektraldichteverteilungsbestimmungseinrichtung (51) andererseits ein Analog-Digital-Umsetzer (59) eingefügt ist, und daß vorzugsweise in die Verbindung zwischen dem zweiten Speicher (56) und dem Monitor (50) ein Digital-Analog-Umsetzer (63) eingefügt ist.

7. Diagnosegerät nach einem der Ansprüche 1 bis 6, **gekennzeichnet** durch eine herzzylklussynchrone Triggerimpulse erzeugende Steuereinrichtung (49), deren Ausgang (66) an die Integriereinrichtung (23), die Spektraldichteverteilungsbestimmungseinrichtung (51) und den ersten Speicher (61) angekoppelt ist und deren Triggerimpulse die zeitlich synchrone Ablaufsteuerung der Integriereinrichtung (23), der Spektraldichteverteilungsbestimmungseinrichtung (51) und des ersten Speichers (61) bewirken.

8. Diagnosegerät nach einem der Ansprüche 1 bis 7, **gekennzeichnet** durch eine die spektrale Verteilungsdichte der Ultraschall-Verschiebungsfrequenzen ermittelnde Frequenzanalysiereinrichtung (Figur 5).

9. Diagnosegerät nach Anspruch 8, **gekennzeichnet** durch eine den Ausgang der Ultraschall-Verschiebungsfrequenz-Meßeinrichtung (19, 20) mit dem Ausgang einer Steuereinrichtung (73) zum Erzeugen eines herzzyklussynchronen Triggerimpulses (92) variabler Zeitdauer verknüpfende Torschaltung (72) und einen an die Torschaltung (72) angekoppelten Mehrkanalanalysator (78 bis 88) zur Mehrkanalanalyse des durch die Torschaltung (72) durchgelassenen Frequenzbereichs der Ultraschall-Verschiebungsfrequenzen.

10. Diagnosegerät nach Anspruch 9, dadurch **gekennzeichnet,** daß die Steuereinrichtung (73) an eine Elektrokardiogrammeinheit (74) angekoppelt ist und in Hintereinanderschaltung folgendes aufweist:
(a) eine Triggerimpulserzeugungsschaltung (75) zum Erzeugen eines herzzyklussynchronen Triggerimpulses (89) vorbestimmter Zeitdauer;
(b) eine Triggerimpulsverzögerungs- oder -verlängerungsschaltung (76) mit einstellbarer Verzögerungs- bzw. Verlängerungsdauer; und
(c) eine durch den verzögerten Triggerimpuls (90) oder die Abstiegsflanke (91) des verlängerten Triggerimpulses (90) getriggerte Impulserzeugungseinrichtung (77), die Impulse (92) einstellbarer Länge erzeugt.

## Claims

1. A diagnosis apparatus for detecting a blood flow signal by measuring the time alterations of the flow of erythrocytes in preterminal and/or terminal vascular systems, comprising:
(a) an ultrasonic shift frequency measuring device (19, 20) for measuring ultrasonic shift frequencies which are caused by the interaction of the erythrocytes with an ultrasonic field,
(b) a device (21) for removing the turbulent flow portion of the erythrocytes from the ultrasonic shift frequency spectrum determined by the ultrasonic shift frequency measuring device (19, 20), which comprises first and second demodulators (32, 33), the first demodulator (32) of which demodulates the electrical shift frequency signals present at the output of an ultrasonic transceiver probe (29), with a first phase shifting, preferably of 0°, in relation to the electric transmitter frequency signal, while the second demodulator (33) demodulates the shift frequency signals present at the output of the ultrasonic transceiver probe (19), with a second phase shifting in relation to the electric transmitter frequency signal, which is preferably 90°;
(c) a filtering device (22) for filtering out a predetermined ultrasonic shift frequency range from the ultrasonic shift frequency spectrum; and
(d) an integrator and comparator device (23) for constituting the integral of the spectral distribution density of the ultrasonic shift frequencies of the predetermined ultrasonic shift frequency range and for comparing the magnitudes of the integrals formed by the integrator and comparator device (23) on the basis of two ultrasonic shift frequency spectrums determined in temporal succession, as well as for providing a diagnosis signal which represents the comparing result;
characterized in that the device (21) for removing the turbulent flow portion of the erythrocytes comprises the following:
(1) a third demodulator (34) which demodulates the shift frequency signals present at the output of the ultrasonic transceiver probe (19), with a third phase shifting in relation to the electric transmitter frequency signal, which is preferably 180°;
(2) a summing device (41) which summarizes the demodulation signals provided by the demodulators (32, 33, 34); and
(3) a comparator device (43) which derives differential signals from the sums of the demodulation signals and the differences of the demodulation signals, and transfers always the largest of these differential signals to its output (42).

2. The diagnosis apparatus according to claim 1, characterized in that the comparator device (43) is a computer.

3. The diagnosis apparatus according to claim 1 or 2, characterized by a monitor (50) which is connected to the integrator and comparator device (23), for displaying the timely course of the difference of the integrals of the spectral distribution density of the ultrasonic shift frequencies of the predetermined ultrasonic shift frequency range.

4. The diagnosis apparatus according to claim 3, characterized in that the monitor (50) is also connected to a spectral distribution density determination device (51), for displaying, preferably simultaneously, the timely course of the spectral distribution density, averaged as regards time, of the ultrasonic shift frequencies of the predetermined ultrasonic shift frequency range, which device comprises two calculators (60, 62) which are connected with their inputs (52, 53) to the output of the filtering device (22) and with their outputs (54, 55) to the input (57, 58) of the integrator and comparator device (23).

5. The diagnosis apparatus according to claim 4, characterized in that the filtering device (22) is connected directly as well as over a first memory (61) to the spectral density distribution determination device (51) and that the spectral density distribution determination device (51) is coupled over a second memory (56) to the integrator and comparator device (23) and the monitor (50).

6. The diagnosis apparatus according to claim 5, characterized in that an analog-to-digital converter (59) is installed in the connection between the filtering device (22) on the one hand and the first memory (61) as well as the spectral density distribution determination device (51) on the other hand, and that it is preferred to insert a digital-to-analog converter (63) in the connection between the second memory (56) and the monitor (50).

7. The diagnosis apparatus according to one of claims 1 to 6, characterized by a control device (49) generating trigger pulses synchronous with the cardiac cycle, the output (66) of which is coupled to the integrator device (23), the spectral density distribution determination device (51) and the first memory (61) and whose trigger pulses effect the timely synchronous sequencing control of the integrator device (23), the spectral density distribution determination device (51) and the first memory (61).

8. The diagnosis apparatus according to one of claims 1 to 7, characterized by a frequency analyzer which determines the spectral distribution density of the ultrasonic shift frequencies (Fig. 5).

9. The diagnosis apparatus according to claim 8, characterized by a gate circuit (72) connecting the output of the ultrasonic shift frequency measuring device (19, 20) to the output of a control unit (73) for generating a trigger pulse (92) of variable duration, which is synchronous with the cardiac cycle, and a multi-channel analyzer (78 to 88) coupled to the gate circuit (72), for multi-channel analyzation of that frequency range of the ultrasonic shift frequencies which passes the gate circuit (72).

10. The diagnosis apparatus according to claim 9, characterized in that the control unit (73) is coupled to an electrocardiogram unit (74) and comprises in series connection the following:
(a) a trigger pulse generation circuit (75) for generating a trigger pulse (89) of a predetermined duration, which is synchronous with the cardiac cycle;
(b) a trigger pulse delay or prolongation circuit (76) with adjustable delay and prolongation duration, respectively; and
(c) a pulse generating device (77) triggered by the delayed trigger pulse (90) or the descent edge (91) of the prolonged trigger pulse (90), which device generates pulses (92) of adjustable duration.

## Revendications

1. Appareil de diagnostic pour détecter un signal de débit sanguin en mesurant la modification dans le temps du débit de globules rouges dans les voies préterminales et/ou terminales d'écoulement du sang, comprenant :
(a) un dispositif de mesure de fréquences de déplacement ultrasonores (19, 20), pour mesurer des fréquences de déplacement ultrasonores qui sont produites par l'interaction des globules rouges avec un champ ultrasonore ;
(b) un dispositif (21) pour supprimer, du spectre de fréquences de déplacement ultrasonores déterminé par le dispositif de mesure de fréquences de déplacement ultrasonores (19, 20), la part d'écoulement turbulent des globules rouges, dispositif qui présente un premier et un deuxième démodulateurs (32, 33), le premier démodulateur (32) démodulant les signaux électriques de fréquences de déplacement, présents à la sortie d'une sonde émettrice et réceptrice d'ultrasons (29), en présence d'un premier déphasage, de préférence de 0°, par rapport au signal électrique de fréquence d'émission, tandis que le deuxième démodulateur (33) démodule les signaux électriques de fréquences de déplacement, présents à la sortie de la sonde émettrice et réceptrice d'ultrasons (29), en présence d'un deuxième déphasage par rapport au signal électrique de fréquence d'émission qui est de préférence égal à 90° ;
(c) un dispositif de filtrage (22) pour supprimer par filtration, du spectre de fréquences de déplacement ultrasonores, une plage prédéterminée de fréquences de déplacement ultrasonores ; et
(d) un dispositif intégrateur et comparateur (23), pour former l'intégrale de la densité de répartition spectrale des fréquences de déplacement ultrasonores de la plage prédéterminée de fréquences de déplacement ultrasonores, et pour comparer quantitativement les intégrales formées par le dispositif intégrateur et comparateur (23) sur la base de deux spectres de fréquences de déplacement ultrasonores déterminés successivement dans le temps, et délivrer un signal de diagnostic représentant le résultat de la comparaison ;
**caractérisé** en ce que le dispositif (21) pour supprimer la part d'écoulement turbulent des globules rouges comprend les éléments suivants :
(1) un troisième démodulateur (34), qui démodule les signaux de fréquences de déplacement, présents à la sortie de la sonde émettrice et réceptrice d'ultrasons (29), en présence d'un troisième déphasage par rapport au signal électrique de fréquence d'émission qui est de préférence égal à 180° ;
(2) un dispositif totalisateur (41), qui additionne les signaux de démodulation délivrés par les démodulateurs (32, 33, 34) ; et
(3) un dispositif comparateur (43), qui forme des signaux différentiels à partir des sommes des signaux de démodulation et des différences des signaux de démodulation, et qui délivre à sa sortie (42) celui de ces signaux différentiels qui est chaque fois le plus grand.

2. Appareil de diagnostic selon la revendication 1, **caractérisé** en ce que le dispositif comparateur (43) est un calculateur.

3. Appareil de diagnostic selon la revendication 1 ou 2, **caractérisé** par un écran de contrôle (50), qui est couplé au dispositif intégrateur et comparateur (23) afin de reproduire l'allure dans le temps de la différence des intégrales de la densité de répartition spectrale des fréquences de déplacement ultrasonores de la plage prédéterminée de fréquences de déplacement ultrasonores

4. Appareil de diagnostic selon la revendication 3, **caractérisé** en ce que l'écran de contrôle (50) est en outre, afin de reproduire de préférence simultanément l'allure dans le temps de la moyenne dans le temps des densités de répartition spectrale des fréquences de déplacement ultrasonores de la plage prédéterminée de fréquences de déplacement ultrasonores, relié à un dispositif de détermination de densité de répartition spectrale (51) qui comprend deux calculateurs (60, 62), qui sont couplés par leur entrée (52, 53) à la sortie du dispositif de filtrage (22) et par leur sortie (54, 55) à l'entrée (57, 58) du dispositif intégrateur et comparateur (23).

5. Appareil de diagnostic selon la revendication 4, **caractérisé** en ce que le dispositif de filtrage (22) est couplé au dispositif de détermination de densité de répartition spectrale (51) à la fois directement et par l'intermédiaire d'une première mémoire (61), et en ce que le dispositif de détermination de densité de répartition spectrale (51) est couplé au dispositif intégrateur et comparateur (23) et à l'écran de contrôle (50) par l'intermédiaire d'une seconde mémoire (56).

6. Appareil de diagnostic selon la revendication 5, **caractérisé** en ce qu'un convertisseur analogique-numérique (59) est intercalé dans la liaison entre le dispositif de filtrage (22) d'une part et la première mémoire (61) ainsi que le dispositif de détermination de densité de répartition spectrale (51) d'autre part, et en ce qu'un convertisseur numérique-analogique (63) est de préférence intercalé dans la liaison entre la seconde mémoire (56) et l'écran de contrôle (50).

7. Appareil de diagnostic selon l'une quelconque des revendications 1 à 6, **caractérisé** par un dispositif de commande (49), qui produit des impulsions de déclenchement synchrones du rythme cardiaque et dont la sortie (66) est couplée au dispositif intégrateur (23), au dispositif de détermination de densité de répartition spectrale (51) et à la première mémoire (61), les impulsions de déclenchement produisant la commande séquentielle, synchrone dans le temps, du dispositif intégrateur (23), du dispositif de détermination de densité de répartition spectrale (51) et de la première mémoire (61).

8. Appareil de diagnostic selon l'une quelconque des revendications 1 à 7, **caractérisé** par un analyseur de fréquences, qui détermine la densité de réparation spectrale des fréquences de déplacement ultrasonores (figure 5).

9. Appareil de diagnostic selon la revendication 8, **caractérisé** par un circuit porte (72) qui combine la sortie du dispositif de mesure de fréquences de déplacement ultrasonores (19, 20) avec la sortie d'un dispositif de commande (73) pour produire une impulsion de déclenchement (92) synchrone du rythme cardiaque, et par un analyseur multicanal (78 à 88), couplé au circuit porte (72) et destiné à l'analyse multicanal de la plage de fréquences de déplacement ultrasonores que laisse passer le circuit porte (72).

10. Appareil de diagnostic selon la revendication 9, **caractérisé** en ce que le dispositif de commande (73) est couplé à une unité d'électrocardiogramme (74) et présente les éléments suivants, montés en série :
(a) un montage générateur d'impulsions de déclenchement (75), pour produire une impulsion de déclenchement (89), synchrone du rythme cardiaque, de durée prédéterminée ;
(b) un montage retardateur ou prolongateur d'impulsions de déclenchement (76), à durée de retardement ou de prolongement réglable ; et
(c) un dispositif générateur d'impulsions (77), qui est déclenché par l'impulsion de déclenchement retardée (90) ou par le flanc de descente (91) de l'impulsion de déclenchement prolongée (90), et qui produit des impulsions (92) de longueur réglable.
